# EUROPEAN PATENT SPECIFICATION

(11) **EP 1 137 383 B1**
(45) Date of publication and mention of the grant of the patent: **20.08.2008**
(21) Application number: 98959402.3
(22) Date of filing: 10.11.1998
(51) Int. Cl.: A61F 13/58

(54) **FASTENER DEVICE**
BEFESTIGUNGSGERÄT
DISPOSITIF DE FIXATION

(43) Date of publication of application: 04.10.2001
(73) Proprietor: THE PROCTER & GAMBLE COMPANY, Cincinnati, Ohio 45202 (US)
(72) Inventor: HAWKINS, Craig, Andrew, Ashiya-shi 659-0087 (JP)
(74) Representative: Borbach, Markus
(86) International application number: PCT/US1998/023887
(87) International publication number: WO 2000/027329

(56) References cited:
- EP-A- 0 324 578
- WO-A-95/11655
- WO-A-97/26851
- US-A- 3 990 449
- US-A- 5 611 789

## Description

### FIELD

The present invention relates to fastener devices. The present invention more particularly relates to a fastener device which has a cover member that protects a securing means from exposure prior to an intended use of the securing means.

### BACKGROUND

Fasteners have previously been used in a variety of disposable and non-disposable articles, including sweat bands, bandages, body wraps, and disposable garments including disposable diapers, and disposable absorbent pads including sanitary napkins and incontinence devices. Fasteners are often provided in such articles and used for joining a part of the article to another member (which may be provided within or outside the articles). The fasteners include a securing means which can mechanically or adhesively secure one member to another member.

One example of application of fasteners is a waist fastening system which is often used in disposable and non-disposable diapers. In this example, the fastener is used for fastening between the front and back ear panels (or side panels) of diapers. Examples of such fasteners are disclosed, for example, in U.S. Patent No. 4,963,140 issued to Robertson et al. on October 16, 1990 and U.S. Patent No. 5,019,065 issued to Scripps on May 28, 1991. Another example of application of fasteners is a fastening system which is used as a disposal securing means for disposable pull-on garments (or diapers) after those have been soiled. In this example, the fastener or disposal device is provided on the outer surface of a backsheet and is used for securing soiled disposable pull-on garments in a convenient disposal configuration. The convenient disposal configuration is preferably achieved by securing a part of the backsheet to the other part of the garments, for example, ear panels (or side panels) through the fastener or disposal device. Examples of such fasteners are disclosed, for example, in WO 98/18421 (Schmitz et al.) published on May 7, 1998; European Patent Publication No. EP0623330 (Hayase et al.) published on November 9, 1994; European Patent Publication No. EP0732094 (Toyoda) published on September 18, 1996; and Japanese Laid-open Patent Publication No. H8-117278 (Tabata) published on May 14. 1996. Yet another example of application of fasteners is a fastening system which is used for securing disposable absorbent pads to wearers' undergarments. In this example, the fastener is provided on the outer surface of a backsheet and is used for securing the absorbent pad to the crotch portion of the wearer's undergarment. Examples of such fasteners are disclosed, for example, in U.S. Patent No. 5,300,058 issued to Goulait et al. on April 5, 1994.

Further fasteners are described in US 5,611,789 and in WO 95/11655. As is noted by the above description, the fasteners are often used in disposable and non-disposable articles in different manners. It is generally expected that the fasteners have a cover member which is provided on the securing means to protect the securing means from being exposed prior to an intended use of the securing means. When the fastener is used, the cover member is removed to expose the securing means. However, such cover member is not completely separatable from the fastener because of a safety issue, especially for infants, i.e., a need for preventing a separated cover member from accidentally going into an infant's month and throat. Another reason is an ease of disposability issue in disposable articles, i.e., if the cover member is connected to the fastener, it can be thrown away together. Thus, the fastener needs to have an un-separatable cover member. However, conventional fasteners which have an un-separatable cover member tend to have a complexity in component member(s) or material(s) used therein, thus tend to be expensive.

Based on the foregoing, there is a need for a fastener which is formed by simple component member(s) or material(s)s.

### SUMMARY

The present invention is directed to a fastener device. The fastener device comprises a base having a first surface and a second surface opposing the first surface; a securing means provided on the first surface of the base; a first adhesive provided on the second surface of the base; and a cover member having an anchor region and a protective region. The anchor region is joined to a part of the second surface of the base through the first adhesive, while the protective region is joined to the securing means so as not to expose the securing means prior to an intended use of the securing means.

The present invention is also directed to a method for making the fastener device.

The foregoing answers the need for a fastener device which is formed by simple component member(s) or material(s)s.

These and other features, aspects, and advantages of the present invention will become evident to those skilled in the art from reading of the present disclosure.

### BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings and which like designations are used to designate substantially identical elements, and in which:
Fig. 1 is a cross-sectional view of a preferred embodiment of the fastener device of the present invention;
Fig. 2 is a cross-sectional view of another preferred embodiment of the fastener device;
Fig. 3 is a cross-sectional view of yet another preferred embodiment of the fastener device;
Fig. 4 is a cross-sectional view of still another preferred embodiment of the fastener device;
Fig. 5 is a cross-sectional view of yet another preferred embodiment of the fastener device;
Fig. 6 is a simplified plan view of a manufacturing process for the fastener device of one embodiment of the present invention;
Fig. 7 is a cross-sectional view of the first continuous member 630 taken along the line 7-7 of Fig. 6;
Fig. 8 is a cross-sectional view of the second continuous member 640 taken along the line 8-8 of Fig. 6;
Fig. 9 is a cross-sectional view of the first and second continuous members 630 and 640 taken along the line 9-9 of Fig. 6;
Fig. 10 is a cross-sectional view of the first and second continuous members 630 and 640 taken along the line 10-10 of Fig. 6; and
Fig. 11 is a cross-sectional view of the first and second continuous members 630 and 640 taken along the line 11-11 of Fig. 6.

### DETAILED DESCRIPTION

All cited references are incorporated herein by reference in their entireties. Citation of any reference is not an admission regarding any determination as to its availability as prior art to the claimed invention.

Herein, "comprise" means that other element(s) and step(s) which do not affect the end result can be added. These terms encompass the terms "consisting of" and "consisting essentially of".

Herein, "disposable garment" describes garments which are not intended to be laundered or otherwise restored or reused as a garment (i.e., they are intended to be discarded after a single use and, preferably, to be recycled, composted or otherwise disposed of in an environmentally compatible manner).

Herein, "joined" or "joining" encompasses configurations whereby an element is directly secured to another by affixing the element directly to the other element, and configurations whereby the element is indirectly secured to the other element by affixing the element to intermediate member(s) which in turn are affixed to the other element.

Herein, "engaging elements" refer to the elements of a hook fastening material which are intended to mechanically engage the fibrous elements of a complementary surface, such as loop fastening materials. The engaging elements may also be referred to as "male" elements.

Herein, "hook" should be non-limiting in the sense that the engaging elements may comprise any shapes as are know in the art so long as they are adapted to engage a complementary surface, such as a loop fastening material or other fibrous material. Examples of useful shapes include, but are not limited to, T-hooks, J-hooks, mushroom, and the like.

Herein, "loop fastening material" means a material having a plurality of fiber elements that are capable of engaging the engaging elements. Such materials are well known to one skilled in the art and include fibrous material, woven and nonwoven. Suitable loop fastening material may be manufactured from a wide range of materials to provide fiber elements, preferably loops. Such suitable materials include nylon, polyester, polypropylene, or any combination of these materials. Suitable examples include, e.g., the commercially available material designated "Scotchmate" brand nylon woven loop No. SJ3401 available from Minnesota Mining and Manufacturing Company, St. Pual, Minnesota, U.S.A.

### A. Structure of Fastener Device

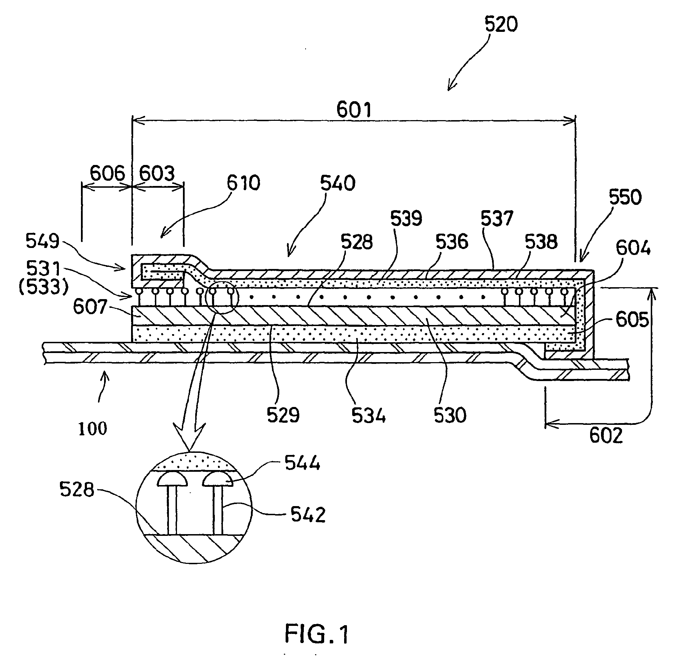

Fig. 1 is a cross-sectional view of a preferred embodiment of the present invention. Referring to Fig. 1, the fastener device 520 includes a base 530 having a first surface 528 and a second surface 529 opposing the first surface 528; a securing means 531 provided on the first surface 528 of the base 530; and an adhesive 534 provided on the second surface 529 of the base 530.

In the embodiment shown in Fig. 1, the fastener device 520 is used in a disposable article 100, for example, a disposable absorbent article (Fig. 1 shows only a part of such disposable article 100 as well as the other Figs.).. The base 530 is joined to a part of the disposable article 100 through the adhesive 534. Preferably, the adhesive 534 is a layer of adhesive for securing at least a part of, more preferably, almost all of the second surface 529 of the base 530 to the disposable article 100. The adhesive 534 is any of those adhesives which provide an adequate bond with the disposable article 100. Preferably, the adhesive 534 is a pressure-sensitive adhesive well-known to those of ordinary skill in the adhesive art.

In a preferred embodiment, the securing means 531 is a hook material which includes first engaging elements 533 which are mechanically engageable with a loop fastening material. The first engaging elements 533 extend from the first surface 528 of the base 530. The first engaging elements 533 may take any configuration as are known by the skilled person in the art so long as they are able to engage a loop fastening material. Each of the engaging elements 533 preferably includes a stem 542 supported at one end on the first surface 528 of the base 530 and a head 544 positioned at the other end of the stem 542. The head 544 can take any shape as are known in the art so long as it is adapted to engage a loop fastening material. In a preferred embodiment, the head 544 has an enlarged shape as shown in Fig. 1.

The first engaging elements 533 may be manufactured from a wide range of materials. Suitable materials include nylon, polyester, polyethylene, polypropylene, or any combination of these materials. Preferred hook materials which include the engaging elements 533 are disclosed in, for example, U.S. Patent 4,894,060 entitled "Disposable Diaper With Improved Hook Fastener Portion" issued to Nestegard on January 16, 1990.

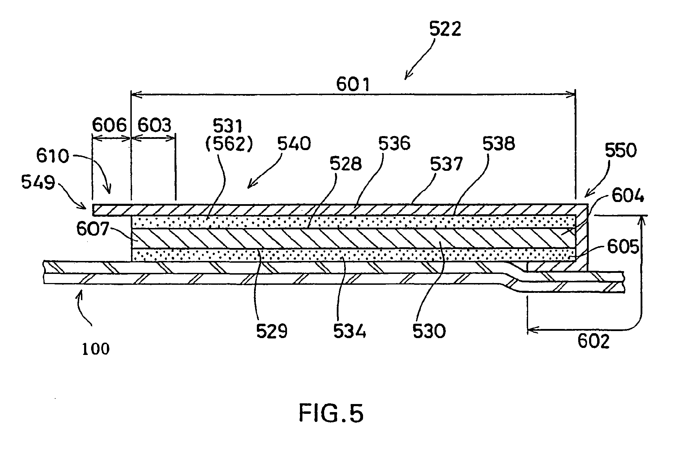

The securing means 531 may be formed by any other material than the hook material, which can be used for securing at least a part of, more preferably, all area of the first surface 528 of the base 530 to an object material to be secured, for example, an adhesive. In a preferred embodiment, an adhesive 562 (not shown in Fig. 1 but Fig. 5) is provided on the first surface 528 of the base 530 as the securing means 531.

The base 530 may be manufactured from a wide range of materials. Suitable materials include nylon, polyester, polyethylene, polypropylene, or any combination of these materials. Preferably, the base 530 is in the form of a thin plastic film of one of these materials. A preferred combined material for the base 530 and the engaging elements 533 is available from The Minnesota Mining and Manufacturing Company, St. Paul, Minn., under Code No. CS-200.

The fastener device 520 of the present invention further includes a cover member 540 which has a protective region 601 and an anchor region 602. Herein, "protective region" refers to a part of the cover member 540 that is positioned on the securing means 531 so as not to expose the securing means 531. Preferably, at least a part of the cover member 540 is joined to the securing means 531 so as not to expose the securing means 531 prior to an intended use of the securing means 531. The anchor region 602 is folded to enclose one edge portion 550 of the base 530 and the securing means 531, and is joined to a part of the second surface 529 of the base 530 through the adhesive 534. This structure provide a hingedly removable feature of the cover member 540 around the one edge portion 550 of the base 530 and the securing means 531. The securing means 531 can be exposed by detaching and removing the protective region 601 of the cover member 540 from the securing means 531 when the user wants to use the securing means 531. The anchor region 602 can be joined to any part of the second surface 529 of the base 530 through the adhesive 534. In a preferred embodiment, the fastener device 520 has the anchor region 602 joined to one end portion 604 of the base 530 through the corresponding end portion 605 of the adhesive 534 as shown in Fig. 1. Preferably, the ratio of the protective region 601 to the anchor region 602 is from about 3:1 to about 10:1, more preferably from about 4:1 to about 5:1.

In a preferred embodiment, the cover member 540 includes a protection member 536 having a first surface 537 and a second surface 538 opposing the first surface 537. The protection member 536 may be manufactured from a wide range of materials. Suitable materials include nylon, polyester, polyethylene, polypropylene, or any combination of these materials. Preferred material for the protection member 536 is in the form of a thin film or a nonwoven of these materials. A suitable combined material for the protection member 536 and the adhesive 539 is available from The Minnesota Mining and Manufacturing Company, St. Paul, Minn., under Code No. KJ-5173L.

More preferably, the cover member 540 further includes an adhesive 539 provided on the second surface 538 of the protection member 536. in a preferred embodiment, the adhesive 539 is a layer of adhesive for securing at least a part of, more preferably, all of the second surface 538 of the protection member 536 to the securing means 531 before the use of the fastener device 520. The adhesive 539 can be any of those adhesives which provide an adequate bond with the securing means 531. Preferably, after the securing member 531 is exposed by removing the cover member 540, the adhesive 539 can work as a secondary or supplemental securing means which can adhesively secure to the object material that is to be secured by the securing means 531. In a preferred embodiment, the adhesive 539 is a pressure-sensitive adhesive well-known to those of ordinary skill in the adhesive art.

In the embodiment shown in Fig. 1, since the fastener device 520 can be formed by uniform component members or materials, i.e., the base 530, the securing means 531, the adhesive 534 and the cover member 540, the manufacturing process for making the fastener device 520 can be simplified. Thus, the cost for the fastener device 520 can be reduced.

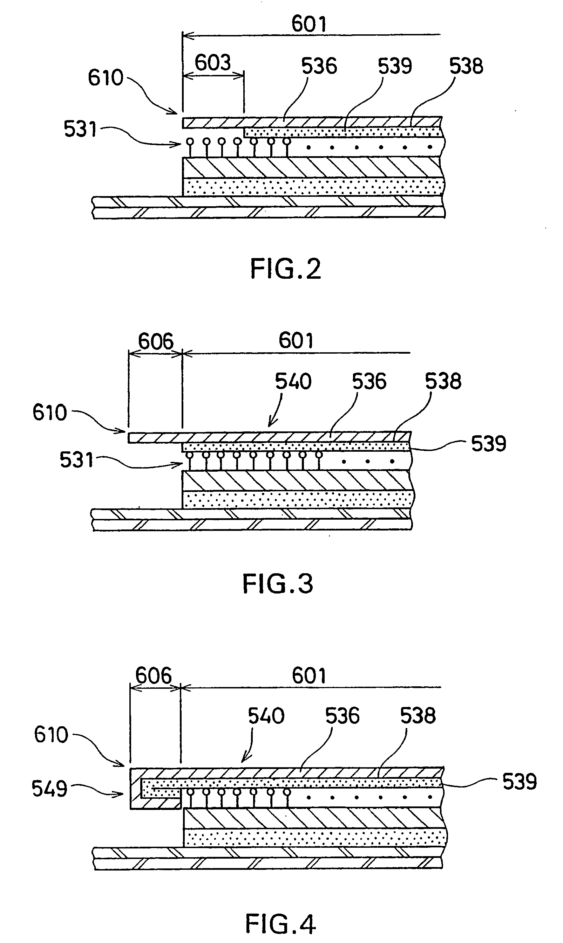

In a preferred embodiment, the cover member 540 further has a tab region 603 which provides a tab portion 610 the user of the fastener device 520 can readily grasp with the fingers when the user wants to remove the cover member 540 to expose the securing means 531. Herein, "tab region" is defined as a part of the protection member 536 which is not joined to the securing means 531. The tab region 603 (thus, the tab portion 610) is formed by not providing an adhesive on a specific area of the second surface 538 of the protection member 536, or by making inactive the adhesive provided on a specific area of the second surface 538 of the protection member 536. In one embodiment, a separate piece of plastic film or nonwoven is attached to cover the adhesive in such specific area to make the adhesive inactive. More preferably, the cover member 540 is folded back onto the second surface 538 of the cover member 540 to make the adhesive inactive as shown in Figs. 1 and 4.

In a preferred embodiment, the tab region 603 is provided within the protective region 601 as shown in Fig. 1. More specifically, while the tab region 603 is positioned within the protective region 601, it is not joined to the securing means 531 thereby forming the tab portion 610. In the embodiment shown in Fig. 1, the tab region 603 is formed by folding the free end portion 549 of the cover member 540 back onto the second surface 538 of the cover member 540. Alternatively, the tab region 603 (thus the tab portion 610) can be formed within the protective region 601 by simply eliminating the adhesive 539 from the second surface 538 of the protection member 536 as shown in Fig. 2.

Yet alternatively, the tab region 603 can be provided at an outside region 606 of the protective region 601. Referring to Fig. 3, the cover member 540 has the outside region 606 which is not joined to the securing means 531 thereby forming the tab portion 610, while the protective region 601 is joined to the securing means 531. As shown in Fig. 3, although the cover member 540 does not have the adhesive 539 on the second surface 538 of the protection member 536 in the outside region 606, the cover member 540 can have the adhesive 539 there for the purpose of a material simplification to be used (not shown in Figs.). In a preferred embodiment, a separate piece of plastic film or nonwoven is attached to the adhesive 539 provided in the outside region 606 to cover the adhesive 539 (not shown in Figs.). In an yet alternative embodiment, the free end portion 549 of the cover member 540 is folded back onto the second surface 538 of the cover member 540 in the outside region 606 thereby forming the tab portion 610 as shown in Fig. 4.

Fig. 5 is a cross-sectional view of another preferred embodiment of the fastener device. Referring to Fig. 5, the fastener device 522 includes a base 530 having a first surface 528 and a second surface 529 opposing the first surface 528; a securing means 531 provided on the first surface 528 of the base 530; and an adhesive 534 provided on the second surface 529 of the bases 530.

In a preferred embodiment wherein this fastener device 522 is used in a disposable article 100 (e.g., a disposable absorbent article), the base 530 is joined to a part of the disposable article 100 through the adhesive 534. Preferably, the adhesive 534 is a layer of adhesive for securing at least a part of, more preferably, almost all of the second surface 529 of the base 530 to the disposable article 100. The adhesive 534 is any of those adhesives which provide an adequate bond with the disposable article 100. Preferably, the adhesive 534 is a pressure-sensitive adhesive well-known to those of ordinary skill in the adhesive art.

In the embodiment shown in Fig. 5, the securing means 531 is an adhesive 562 provided on the first surface 528 of the base 530. Preferably, the adhesive 562 is a layer of adhesive for adhesively securing at least a part of, more preferably, all area of the second surface 528 of the base 530 to an object material, for example, a plastic film, a nonwoven, and the like (not shown in Figs.). In a preferred embodiment, the adhesive 562 is a pressure-sensitive adhesive well-known to those of ordinary skill in the adhesive art.

The base 530 may be manufactured from a wide range of materials. Suitable materials include nylon, polyester, polyethylene, polypropylene, or any combination of these materials. Preferably, the base 530 is in the form of a thin film of one of these materials. A preferred combined material for the base 530, the adhesives 531 and 534 is available from The Minnesota Mining and Manufacturing Company, St. Paul, Minn., under Code No. LS-200.

The fastener device 520 of the present invention further includes a cover member 540 which has a protective region 601 and an anchor region 602. The cover member 540 has the protective region 601 positioned on the securing means 531. The protective region 601 is joined to the securing means 531 so as not to expose the securing means 531 prior to an intended use of the securing means 531 (i.e., the adhesive 562). The anchor region 602 is folded to enclose one edge portion 550 of the base 530 and the securing means 531, and is joined to a part of the second surface 529 of the base 530 through the adhesive 534. This structure provides a hingedly removable feature of the cover member 540 around the one edge portion 550 of the base 530 and the securing means 531. The securing means 531 can be exposed by detaching and removing the protective region 601 of the cover member 540 from the securing means 531 when the user wants to use the securing means 531. The anchor region 602 can be joined to any part of the second surface 529 of the base 530 through the adhesive 534. In a preferred embodiment, the fastener device 522 has the anchor region 602 joined to one end portion 604 of the base 530 through the corresponding end portion 605 of the adhesive 534 as shown in Fig. 5. Preferably, the ratio of the protective region 601 to the anchor region 602 is from about 3:1 to about 10:1, more preferably from about 4:1 to about 5:1.

In a preferred embodiment, the cover member 540 includes a protection member 536 having a first surface 537 and a second surface 538 opposing the first surface 537. The protection member 536 may be manufactured from a wide range of materials. Suitable materials include nylon, polyester, polyethylene, polypropylene, or any combination of these materials. Preferred material for the protection member 536 is in the form of a thin film or a nonwoven of these materials. A suitable combined material for the protection member 536 and the adhesive 539 is available from The Minnesota Mining and Manufacturing Company, St. Paul, Minn., under Code No. KJ-5173L.

In a preferred embodiment, the cover member 540 further has a tab region 603 which provides a tab portion 610 the user of the fastener device 522 can readily grasp with the fingers when the user wants to remove the cover member 540 to expose the securing means 531. The tab portion 610 is formed by not providing an adhesive on a specific area of the first surface 528 of the base 530, or by making inactive the adhesive provided on a specific area of the first surface 528 of the base 530. In one embodiment, a separate piece of plastic film or nonwoven is attached to cover the adhesive in such specific area to make the adhesive inactive.

In the embodiment shown in Fig. 5, the tab region 603 is provided at an outside region 606 of the protective region 601. More specifically, the cover member 540 has the outside region 606 which is not joined to the securing means 531 thereby forming the tab portion 610, while the protective region 601 is joined to the securing means 531.

In an alternative preferred embodiment, the tab region 603 is provided within the protective region 601 (not shown in Figs.). More specifically, while the tab region 603 is positioned within the protective region 601, the adhesive 562 is eliminated in the tab region 603. Thus, the protection member 536 is not joined to the securing means 531 in the tab region 603 thereby forming the tab portion 610.

### B. Method for Making Fastener Device

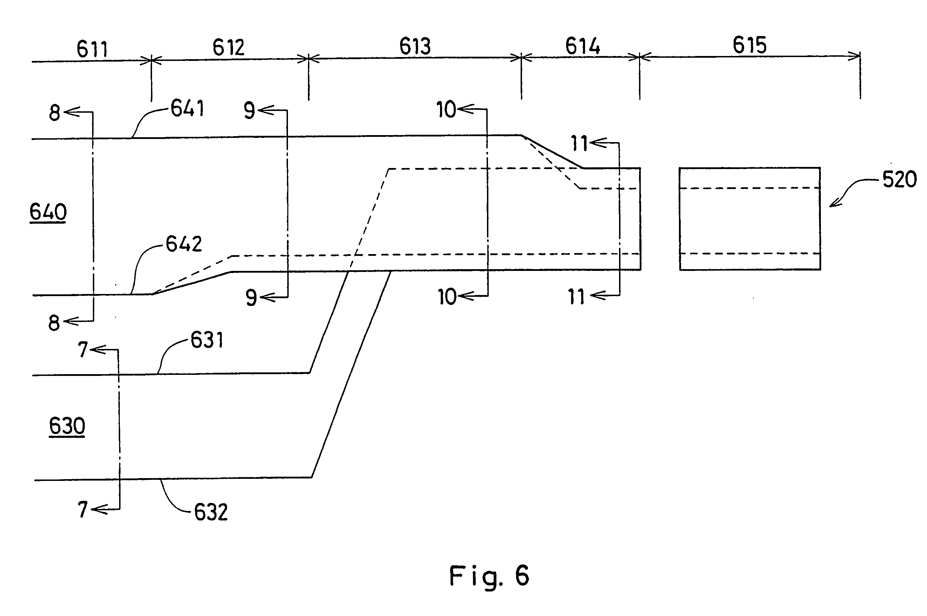

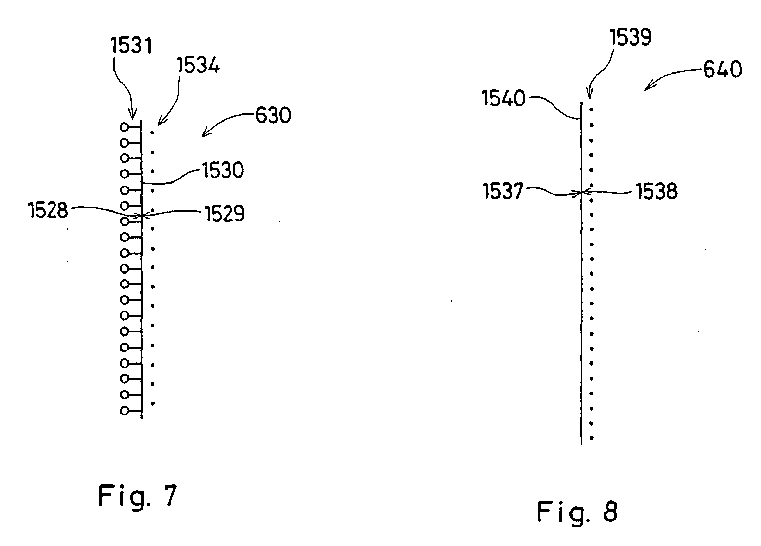

Fig. 6 is a simplified plan view of a manufacturing process for the fastener device 520 shown in Fig. 1. Each of Figs. 7 to 11 is a cross-sectional view of the first continuous member 630 and/or the second continuous member 640 taken along the respective lines 7-7 to 11-11 of Fig. 6. The fastening device 520 according to the present invention can be manufactured by the following steps.

Referring to Fig. 6, in Step 611, a first continuous member 630 which has a first side edge portion 631 and a second side edge portion 632 opposing the first side edge portion 631 is provided. Referring to Fig. 7, the first continuous member 630 includes (a) a base layer 1530 having a first surface 1528 and a second surface 1529 opposing the first surface 1528, (b) a securing means layer 1531 provided on the first surface 1528 of the base layer 1530, and (c) an adhesive layer 1534 provided on the second surface 1529 of the base layer 1530. In a preferred embodiment, the first continuous member 630 is formed by a uniform member which includes the base layer 1530, the securing means layer 1531 provided on the first surface 1528 of the base layer 1530, and the adhesive layer 1534 provided on the second surface 1529 of the base layer 1530. Herein, "uniform member" means a member being formed by, at any location through the member, a substantially same structure employing substantially identical component materials.

Referring again to Fig. 6, in Step 611, a second continuous member 640 which has a first side edge portion 641 and a second side edge portion 642 opposing the first side edge portion 641 is also provided. Referring to Fig. 8, the second continuous member 640 includes a cover member layer 1540 having a first surface 1537 and a second surface 1538 opposing the first surface 1537. Preferably, the second continuous member 640 further includes an adhesive layer 1539 provided on the second surface 1538 of the cover member layer 1540. In a preferred embodiment, the second continuous member 640 is formed by a uniform member which is formed by the cover member layer 1540 and the adhesive layer 1539 provided on the second surface 1538 of the cover member layer 1540.

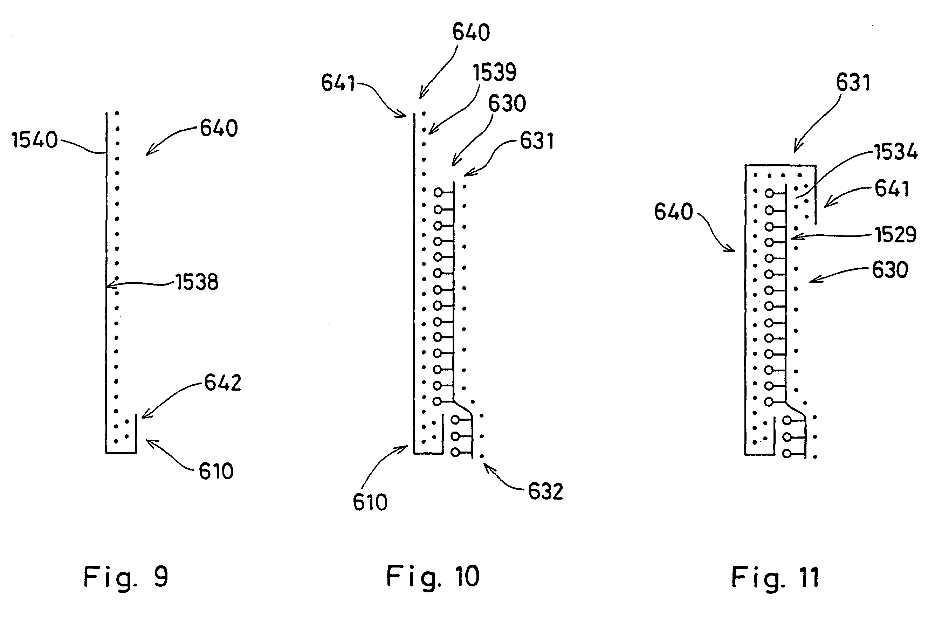

In a preferred embodiment, in Step 612 of Fig. 6, the second side edge portion 642 of the second continuous member 640 is folded back onto the second surface 1538 of the cover member layer 1540 to make a tab portion 610 which is shown in Fig. 9.

In Step 613, the first and second continuous members 630 and 640 are juxtaposed and joined together through the adhesive layer 1539 such that the side edge portion 641 of the second continuous member 640 is projected compared with the side edge portion 631 of the first continuous member 630 as shown in Fig. 10. In the embodiment shown in Fig. 10, the side edge portion 632 of the first continuous member 630 is aligned with the tab portion 610 which was formed by the second continuous member 640.

In Step 614, the side edge portion 641 of the second continuous member 640 is folded to enclose the side edge portion 631 of the first continuous member 630, and fixed to the second surface 1529 of the first continuous member 630 through the adhesive layer 1534 as shown in Fig. 11.

In Step 615, the resulting continuous member which is comprised of the first and second continuous members 630 and 640 is cut at a predetermined length by a cutting means (not shown in Figs.) which is well known in the art, thereby producing an individual fastener device 520.

In a preferred embodiment wherein the fastener device 520 is used for a disposal garment, the fastener device 520 is joined to a part of the disposal garment (e.g., the outer-facing surface of a backsheet) through the adhesive layer 1534 after Step 615 (not shown in Figs.).

### C. Application of Fastener Device to Disposable Article

The fastener device of the present invention can be applied to a variety of articles (disposable or re-useable) in need of a fastener. Preferred disposable articles include sweat bands, bandages, body wraps, disposable diapers (adult and baby), and disposable absorbent pads including sanitary napkins. In a preferred embodiment, the disposable article is a disposable garment which includes a liquid impervious backsheet, and the fastener device is joined to a part of the backsheet.

In a preferred embodiment, the fastener device 520 (shown in Fig. 1, for example) is used in a disposable pull-on garment (e.g., a pull-on diaper) as a disposal device. After a disposable pull-on garment is soiled by excreta, it is folded to contain the contents within the soiled pull-on garment. The folded garment is secured by the fastener device to prevent the contents in the soiled pull-on garment from leaking out from the soiled pull-on garment.

The disposable pull-on garment has a front region, a back region and a crotch region between the front region and the back region. The disposable pull-on garment includes a chassis provided in the front, back and crotch regions. The chassis includes a liquid pervious topsheet, a liquid impervious backsheet associated with the topsheet, and an absorbent core disposed between the topsheet and the backsheet. The disposable pull-on garment further includes at least one pair of ear panels extending laterally outwardly from the chassis in the front or back region. The ear panels are joined to the chassis to form two leg openings and a waist opening. The disposable pull-on garment further includes the fastener device of the present invention joined to the backsheet. The fastener device can be joined to any place in the backsheet as long as the disposable pull-on garment can be secured by the fastener device in a configuration that provides a convenient disposal after the disposable pull-on garment has been soiled. Preferably, the fastener device is joined to the backsheet in the crotch region. Examples of suitable locations for joining the fastener device of the present invention are disclosed, for example, in WO 98/18421 (Schmitz et al.) published on May 7, 1998 and Japanese Laid-open Patent Publication No. H8-117278 (Tabata) published on May 14, 1996, wherein one would substitute the fastener disclosed therein with the fastener device of the present invention.

In one embodiment, the fastener device 520 (shown in Fig. 1, for example) is used for a waist fastening system in a disposable garment (e.g., a tape type diaper). In this embodiment, the fastener device is used for the fastening between a front and back ear panels (or side panels) of the disposable garment.
The disposable garment has a front region, a back region and a crotch region between the front region and the back region. The disposable garment includes a chassis provided in the front, back and crotch regions. The chassis includes a liquid pervious topsheet, a liquid impervious backsheet associated with the topsheet, and an absorbent core disposed between the topsheet and the backsheet. The disposable garment further includes at least one pair of ear panels extending laterally outwardly from the chassis in the front or back region. The ear panels are joined to the chassis. The disposable garment further includes the fastener devices of the present invention each joined to the respective ear panel. The fastener device can be joined to any place in the ear panel as long as the disposable garment can be secured by the fastener device around the waist of the wearer. Preferably, the fastener device is provided adjacent to the longitudinal edge portion of the ear panel. Examples of suitable locations for joining the fastener device of the present invention are disclosed, for example, in U.S. Patent No. 4,963,140 issued to Robertson et al. on October 16, 1990 and U.S. Patent No. 5,019,065 issued to Scripps on May 28, 1991, wherein one would substitute the fastener disclosed therein with the fastener device of the present invention.

In an alternative embodiment, the fastener device can be provided on the backsheet in the front region as a part of a fastener system. In this embodiment, the disposable garment can be secured around the waist of the wearer by joining the ear panel to the backsheet through the fastener device. Preferably, the fastener device is provided adjacent to the waist edge of the chassis.

In yet another embodiment, the fastener device 520 (shown in Fig. 1, for example) is used for a fastener for securing a disposable absorbent pad (e.g., a sanitary napkin, a pantiliner and an incontinent pad) to the crotch of a wearer's undergarment. In this embodiment, the fastener device is provided on the outer surface of a backsheet.

The disposable absorbent pad includes a liquid pervious topsheet, a liquid impervious backsheet associated with the topsheet, and an absorbent core disposed between the topsheet and the backsheet. The fastener device can be joined to any place in the ear panel as long as the disposable absorbent pad can be secured by the fastener device to the crotch of a wearer's undergarment. Examples of suitable locations for joining the fastener device of the present invention are disclosed, for example, in U.S. Patent No. 5,300,058 issued to Goulait et al. on April 5, 1994, wherein one would substitute the fastener disclosed therein with the fastener device of the present invention.

It is understood that the examples and embodiments described herein are for illustrative purpose only and that various modifications or changes will be suggested to one skilled in the art without departing from the scope of the present invention.

## Claims

1. A fastener device (520) comprising:
a base (530) having a first surface (528) and a second surface (529) opposing the first surface (528);
a securing means (531) provided on the first surface (528) of the base (530);
a first adhesive (534) provided on the second surface (528) of the base (530); and
a cover member (540) having an anchor region (602) and a protective region (601), wherein the anchor region (602) is joined to a part of the second surface (529) of the base (530) through the first adhesive (534) and the protective region (601) is joined to the securing means (531) so as not to expose the securing means prior to an intended use of the securing means (531),

2. The fastener device (520) of Claim 1, wherein the securing means (531) is a second adhesive provided on the first surface (528) of the base (530).

3. The fastener device (520) of Claim 1. wherein the securing means (531) is a plurality of engaging elements extending from the first surface of the base (530), and wherein the engaging elements are mechanically engageable with a loop fastening material.

4. The fastener device (520) of Claim 3, further comprising an adhesive provided between the cover member (540) and the engaging elements.

5. The fastener device (520) of Claim 1, wherein the cover member (540) further has a tab region (603) which forms a tab portion (610).

6. A disposable article comprising the fastener device of Claim 1

7. The disposable article of Claim 6, wherein the disposable article is a disposable garment which includes a liquid impervious backsheet, and wherein the fastener device is joined to a part of the backsheet.

8. The disposable article of Claim 6, wherein the disposable article is a disposable garment which includes a waist fastener system employing the fastener device of Claim 1.

9. The disposable article of Claim 7, wherein the disposable absorbent garment is a diaper, an incontinent pad, a sanitary napkin, or a pantiliner.

10. A method for making a fastener device (520) comprising the steps of:
providing a first continuous member (630) having two side edge portions (631,632), the first continuous member (630) including (a) a base layer (1530) having a first surface (1528) and a second surface (1529) opposing the first surface (1528), (b) a securing means provided on the first surface (1528) of the base layer (1530), and (c) a first adhesive layer (1534) provided on the second surface (1529) of the base layer (1530);
providing a second continuous member (640) having two side edge portions (641, 642), the second continuous member (640) including a cover layer (1540) having a first surface (1537) and a second surface (1538) opposing the first surface (1537);
juxtaposing the first and second continuous members (630,640) such that one of the two side edge portions (641,642) of the second continuous member (640) is projected compared with the corresponding one side edge portion of the first continuous member (630);
fixing the projected side edge portion of the second continuous member (640) to the second surface (1529) of the first continuous member (630) through the first adhesive (1534); and
cutting the first and second continuous members (630, 640) at a predetermined width to form a fastener device (520).

## Patentansprüche

1. Befestigungsvorrichtung (520), aufweisend:
eine Basis (530) mit einer ersten Oberfläche (528) und einer zweiten Oberfläche (529), die der ersten Oberfläche (528) gegenüber liegt;
ein Sicherungsmittel (531), das auf der ersten Oberfläche (528) der Basis (530) vorgesehen ist;
ein erstes Haftmittel (534), das auf der zweiten Oberfläche (528) der Basis (530) vorgesehen ist; und
ein Abdeckungselement (540) mit einer Ankerregion (602) und einer Schutzregion (601), wobei die Ankerregion (602) durch das erste Haftmittel (534) an einen Teil der zweiten Oberfläche (529) der Basis (530) gefügt ist, und die Schutzregion (601) an das Sicherungsmittel (531) gefügt ist, damit das Sicherungsmittel (531) vor einer beabsichtigten Verwendung des Sicherungsmittels (531) nicht freigelegt wird.

2. Befestigungsvorrichtung (520) nach Anspruch 1, wobei das Sicherungsmittel (531) ein zweites Haftmittel ist, das auf der ersten Oberfläche (528) der Basis (530) vorgesehen ist.

3. Befestigungsvorrichtung (520) nach Anspruch 1, wobei es sich bei dem Sicherungsmittel (531) um eine Vielzahl von Eingriffselementen handelt, die von der ersten Oberfläche der Basis (530) vorstehen, und wobei die Eingriffselemente mechanisch mit einem Schlaufenbefestigungsmaterial in Eingriff gebracht werden können.

4. Befestigungsvorrichtung (520) nach Anspruch 3, ferner ein Haftmittel umfassend, das zwischen dem Abdeckungselement (540) und den Eingriffselementen vorgesehen ist.

5. Befestigungsvorrichtung (520) nach Anspruch 1, wobei das Abdeckungselement (540) ferner eine Laschenregion (603) bildet, die einen Laschenabschnitt (610) bildet.

6. Einweg-Artikel, die Befestigungsvorrichtung nach Anspruch 1 umfassend.

7. Einweg-Artikel nach Anspruch 6, wobei der Einweg-Artikel ein Einwegkleidungsstück ist, das eine flüssigkeitsundurchlässige untere Lage einschließt, und wobei die Befestigungsvorrichtung an einen Teil der unteren Lage angefügt ist.

8. Einweg-Artikel nach Anspruch 6, wobei der Einweg-Artikel ein Einwegkleidungsstück ist, das ein Taillenbefestigungssystem einschließt, das die Befestigungsvorrichtung nach Anspruch 1 verwendet.

9. Einweg-Artikel nach Anspruch 7, wobei es sich bei dem absorbierenden Einweg-Absorptionsartikel um eine Windel, eine Inkontinzenzeinlage, eine Damenbinde oder eine Slipeinlage handelt.

10. Verfahren zur Herstellung einer Befestigungsvorrichtung (520), die folgenden Schritte umfassend:
Bereitstellen eines ersten kontinuierlichen Elements (630) mit zwei Seitenrandabschnitten (631, 632) wobei das erste kontinuierliche Element (630) (a) eine Basisschicht (1530) mit einer ersten Oberfläche (1528) und einer zweiten Oberfläche (1529) gegenüber der ersten Oberfläche (1528), (b) ein Sicherungsmittel, das auf der ersten Oberfläche (1528) der Basisschicht (1530) vorgesehen ist, und (c) eine erste Haftmittelschicht (1534), die auf der zweiten Oberfläche (1529) der Basisschicht (1530) vorgesehen ist, aufweist;
Bereitstellen eines zweiten kontinuierlichen Elements (640) mit zwei Seitenrandabschnitten (641, 642), wobei das zweite kontinuierliche Element (640) eine Abdeckungsschicht (1540) mit einer ersten Oberfläche (1537) und einer zweiten Oberfläche (1538) gegenüber der ersten Oberfläche (1537) aufweist;
Anordnen der ersten und zweiten kontinuierlichen Elemente (630, 640) einander gegenüber, so dass einer der beiden Seitenrandabschnitte (641, 642) des zweiten kontinuierlichen Elements (640) im Vergleich zum entsprechenden Seitenrandabschnitt des ersten kontinuierlichen Elements (630) übersteht;
Fixieren des überstehenden Seitenrandabschnitts des zweiten kontinuierlichen Elements (640) an der zweiten Oberfläche (1529) des ersten kontinuierlichen Elements (630) durch das erste Haftmittel (1534); und
Schneiden der ersten und zweiten kontinuierlichen Elemente (630, 640) auf eine vorgegebene Breite, um eine Befestigungsvorrichtung (520) zu bilden.

## Revendications

1. Dispositif de fixation (520) comprenant :
une base (530) présentant une première surface (528) et une deuxième surface (529) opposée à la première surface (528) ;
un moyen d'attache (531) fourni sur la première surface (528) de la base (530) ;
un premier adhésif (534) fourni sur la deuxième surface (528) de la base (530) ; et
un élément de protection (540) ayant une zone d'ancrage (602) et une zone de protectrice (601), dans lequel la zone d'ancrage (602) est assemblée à une partie de la deuxième surface (529) de la base (530) par l'intermédiaire du premier adhésif (534) et la zone protectrice (601) est attachée au moyen d'attache (531) de manière à ne pas exposer le moyen d'attache (531) avant une utilisation intentionnelle du moyen d'attache (531).

2. Dispositif de fixation (520) selon la revendication 1, dans lequel le moyen d'attache (531) est un deuxième adhésif fourni sur la première surface (528) de la base (530).

3. Dispositif de fixation (520) selon la revendication 1, dans lequel le moyen d'attache (531) est une pluralité d'éléments d'accrochage s'étendant à partir de la première surface de la base (530), et dans lequel les éléments d'accrochage peuvent être accrochés mécaniquement avec un matériau de fixation à boucles.

4. Dispositif de fixation (520) selon la revendication 3, comprenant, en outre, un adhésif fourni entre l'élément de protection (540) et les éléments d'accrochage.

5. Dispositif de fixation (520) selon la revendication 1, dans lequel l'élément de protection (540) comprend, en outre, une région à languette (603) qui forme une partie de languette (610).

6. Article jetable comprenant le dispositif de fixation selon la revendication 1.

7. Article jetable selon la revendication 6, où l'article jetable est un vêtement jetable qui comprend une feuille de fond imperméable aux liquides, et dans lequel le dispositif de fixation est attaché à une partie de la feuille de fond.

8. Article jetable selon la revendication 6, où l'article jetable est un vêtement jetable qui comprend un dispositif d'attache de la taille utilisant le dispositif de fixation selon la revendication 1.

9. Article jetable selon la revendication 7, dans lequel le vêtement absorbant jetable est une couche, une serviette d'incontinence, une serviette hygiénique ou un protège-slip.

10. Procédé pour fabriquer un dispositif de fixation (520), comprenant les étapes consistant à :
fournir un premier élément continu (630) ayant deux parties de bord latéral (631, 632), le premier élément continu (630) incluant (a) une couche de base (1530) ayant une première surface (1528) et une deuxième surface (1529) opposée à la première surface (1528), (b) un moyen d'attache fourni sur la première surface (1528) de la couche de base (1530), et (c) une première couche adhésive (1534) fournie sur la deuxième surface (1529) de la couche de base (1530) ;
fournir un deuxième élément continu (640) ayant deux parties de bord latéral (641, 642), le deuxième élément continu (640) incluant une couche de protection (1540) ayant une première surface (1537) et une deuxième surface (1538) opposée à la première surface (1537) ;
juxtaposer le premier et le deuxième éléments continus (630, 640) de telle sorte qu'une des deux parties de bord latéral (641, 642) du deuxième élément continu (640) est projetée par rapport à la partie de bord latéral correspondante du premier élément continu (630) ;
fixer la partie de bord latéral projetée du deuxième élément continu (640) sur la deuxième surface (1529) du premier élément continu (630) par l'intermédiaire du premier adhésif (1534) ; et
couper le premier et le deuxième éléments continus (630, 640) à une largeur prédéterminée pour former un dispositif de fixation (520).
